# EUROPÄISCHE PATENTSCHRIFT

(11) **EP 0 633 039 B1**
(45) Veröffentlichungstag und Bekanntmachung des Hinweises auf die Patenterteilung: **13.10.2004**
(21) Anmeldenummer: 94110322.8
(22) Anmeldetag: 02.07.1994
(51) Int. Cl.: A61M 39/10

(54) **Bajonett-Schlauchverbinder**
Bayonet connector for tubes
Connecteur à bayonnette pour tubes

(30) Priorität: 09.07.1993 DE 4322868
(43) Veröffentlichungstag der Anmeldung: 11.01.1995
(73) Patentinhaber: B. BRAUN MELSUNGEN AG, 34212 Melsungen (DE)
(72) Erfinder: Eick, Rüdiger, D-66459 Kirkel (DE); Mehner, Gotthilf, D-66280 Sulzbach (DE); Sachers, Klaus-Peter, D-67550 Worms (DE)
(74) Vertreter: Selting, Günther, Dipl.-Ing.

(56) Entgegenhaltungen:
- WO-A-93/08870
- DE-A- 1 766 739
- DE-U- 8 529 783
- US-A- 4 187 846

## Beschreibung

### Technisches Gebiet

Die Erfindung bezieht sich auf einen Schlauchverbinder für medizinische Zwecke.

### Stand der Technik

In der Medizintechnik besteht häufig das Problem des Verbindens von Schlauchleitungen, beispielsweise im Zusammenhang mit Drainagen, insbesondere Vakuumdrainagen (Drainage nach Redon). Von solchen Schnittstellen ist zu fordern, daß Konnektieren wie Dekonnektieren einfach, sicher und mit möglichst geringem Kraftaufwand erfolgen soll. Da solche Schnittstellen in der Regel Einmalartikel sind, sollte der Schlauchverbinder möglichst kostengünstig herstellbar sein, ohne gegenüber präziser zu fertigenden und damit teureren Mehrmalartikeln bezüglich der Funktionalität Nachteile aufzuweisen.

In der Medizintechnik haben für solche Schnittstellen sog. Luer-Lock-Konnektoren weite Verbreitung gefunden. Ein Luer-Lock-Konnektor besteht aus einem weiblichen Teil mit außenseitigem Gewinde und konischer Aufnahmeöffnung sowie einem entsprechenden konischen männlichen Teil mit einer Überwurfmutter mit Innengewinde zum Verschrauben mit dem Außengewinde des weiblichen Teils. Beim Betätigen der Verbindungsvorrichtung durch Verschrauben der beiden Teile werden die Oberflächen des Innenkonus des weiblichen Teils und des Außenkonus des männlichen Teils formschlüssig aufeinandergepreßt und so eine Dichtwirkung erzielt. Nachteilig an diesen Konnektoren ist die Abhängigkeit der Dichtigkeit von der beim Zusammenschrauben aufgewendeten Kraft. Bei zu lockerem Zusammenschrauben besteht die Gefahr der Undichtigkeit. Andererseits besteht bei zu kräftigem Zusammenschrauben die Gefahr der Zerstörung des Konnektors. Außerdem besteht insbesondere beim Verbinden relativ starrer Schläuche die Gefahr, daß sich durch die durch das Verwinden des Schlauches beim Zusammenschrauben der beiden Teile des Luer-Lock-Konnektors oder beim Hantieren mit den verbundenen Schläuchen entstehenden Rückstellkräfte die Verbindung lockert oder gar löst. Durch das formschlüssige Aufeinanderpressen der beiden Konnektorteile besteht außerdem die Gefahr des Verklebens. Bedingt durch Quellung der Bauteile in Gegenwart von Flüssigkeiten wird außerdem das Auftreten von Spannungsrissen beobachtet.

### Beschreibung der Erfindung

Aufgabe der vorliegenden Erfindung ist es, einen Schlauchverbinder für medizinische Zwecke zur Verfügung zu stellen, der kostengünstig zu fertigen ist, einfaches mit geringem Kraftaufwand zu bewerkstelligendes Konnektieren und Dekonnektieren erlaubt, nicht zum Verkleben neigt, unerwünschtes Dekonnektieren sicher vermeidet und nicht anfällig für das Auftreten von Spannungsrissen ist.

Aus der WO-A 93/08870, die zur Abgrenzung des Anspruchs herangezogen worden ist, ist ein Kupplungsstück zum Verbinden von Kühlmittelschläuchen bekannt, bei dem das weibliche Kupplungsstück und das männliche Kupplungsstück jeweils eine Nut mit einem Dichtring aufweisen. Beim Zusammenschrauben der beiden Kupplungsstücke bildet sich zwischen den beiden Dichtringen eine geschlossene Kammer. Durch Leckage in die Kammer eingedrungenes Kühlmittel erhöht den Druck in der Kammer und verhindert dadurch weitere Leckage.

Es wurde nun gefunden, daß die Aufgabe hervorragend gelöst wird durch einen Schlauchverbinder für Vakuumdrainagen, bestehend aus einem weiblichen Kupplungsteil mit Innenkonus, einem männlichem Kupplungsteil mit Außenkonus mit gleicher Konizität, wobei der Außenkonus eine Nut mit einem elastischen Dichtring aufweist, und aus einer Verbindungseinrichtung, der dadurch gekennzeichnet ist, daß als Verbindungseinrichtung ein Bajonett-Verschluß vorgesehen ist, der durch zwei Bajonett-Zapfen am männlichen Kupplungsteil, die mit zwei Bajonett-Kurvennuten des weiblichen Kupplungsteiles korrespondieren, gebildet wird, und der elastische Dichtring die Dichtwirkung zwischen den beiden Kupplungsteilen in verbundenem Zustand bewirkt, und der Bajonettverschluß so dimensioniert ist, daß beim Verbinden der Kupplungsteile zwischen Innenkonus und Außenkonus ein definierter Spalt entsteht.

Der erfindungsgegenständliche Schlauchverbinder wird nach den dem Fachmann geläufigen Verfahren aus den in der Medizintechnik für solche Gegenstände üblichen Materialien hergestellt.

Ein Ausführungsbeispiel der Erfindung wird anhand der folgenden Zeichnungen beschrieben:

Es zeigen
- Fig. 1: die Einzelteile des erfindungsgemäßen Schlauchverbinders im Längsschnitt
- Fig. 2: die Einzelteile des erfindungsgemäßen Schlauchverbinders in Aufsicht zu Fig. 1
- Fig. 3: den Schlauchverbinder im gekuppelten Zustand und Betriebsstellung
- Fig. 4: eine Darstellung entsprechend Fig. 3 in Aufsicht.

In den Zeichnungen ist mit PN die patientennahe Seite und mit PF die patientenferne Seite des Schlauchverbinders bezeichnet.

Der Schlauchverbinder 1 besteht aus den beiden Hauptteilen 2 und 3, wobei das Hauptteil 2 das sog. weibliche Kupplungsteil darstellt, während das Hauptteil 3 das sog. männliche Kupplungsteil darstellt.

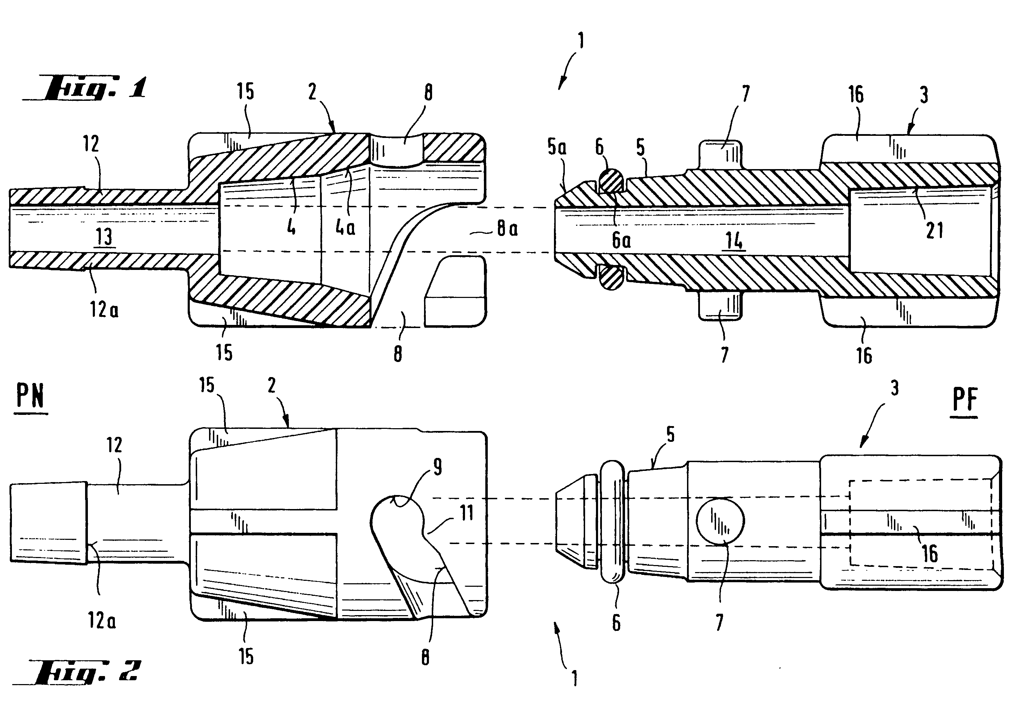

Das weibliche Kupplungsteil 2 weist einen Innenkonus 4 auf. Das männliche Kupplungsteil 3 weist einen Außenkonus 5 mit gleicher Konizität auf. Am Außenkonus 5 ist eine Nut 6a zur Aufnahme eines Dichtungsringes 6 vorgesehen. Zur Erleichterung des Zusammenfügens beider Kupplungsteile sind Einführkonen 4a bzw. 5a an den Kupplungsteilen 2 und 3 vorgesehen. Das männliche Kupplungsteil 3 weist zwei Bajonett-Zapfen 7 auf, welche mit zwei Bajonett-Kurven-Nuten 8 des weiblichen Kupplungsteiles 2 korrespondieren. Wie insbesondere Fig. 2 veranschaulicht, ist in einer Entfernung, welche dem Durchmesser des Bajonett-Zapfens 7 entspricht, vor dem Ende 9 der Bajonett-Kurven-Nut 8 eine Rastnocke 11 angeordnet.

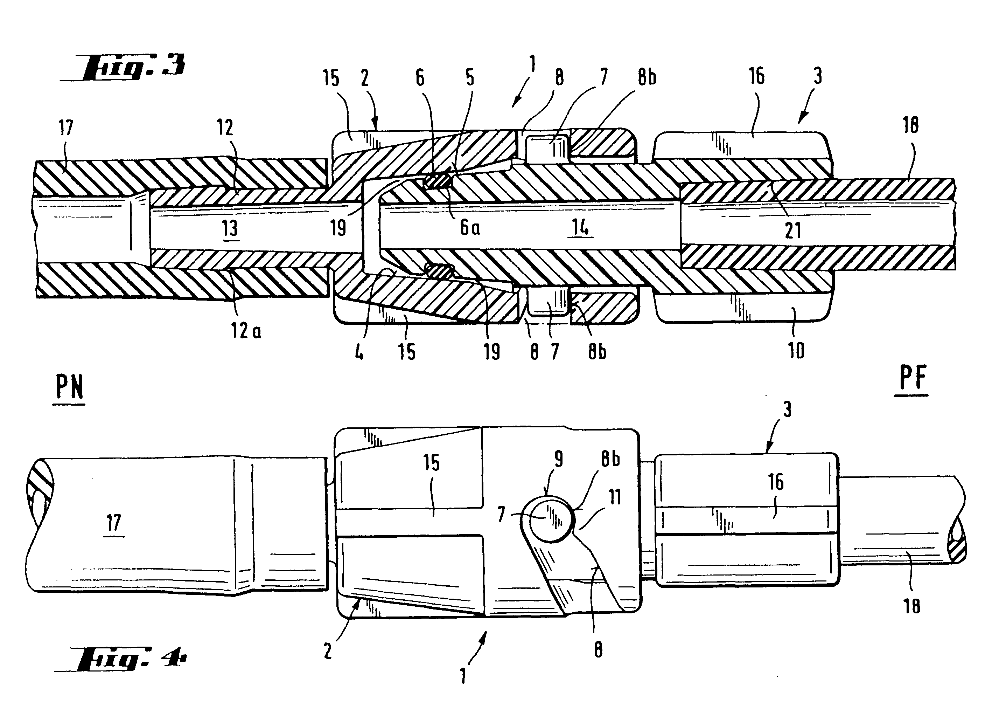

Die beiden Kupplungsteile werden dermaßen miteinander verbunden, daß der Konus 5 in den Konus 4 bei einer Position eingeführt wird, in welcher sich die Bajonett-Zapfen 7 mit den Eingängen 8a zu den Kurven-Nuten 8 axial überdecken. Anschließend wird das männliche Kupplungsteil 3 gegenüber dem weiblichen Kupplungsteil 2 nach rechts verdreht. Dabei kommen die schraubenförmig verlaufenden Bajonett-Kurven-Nuten 8 axial spannend zur Wirkung, was zur Kompression des Dichtungsringes 6 führt (Fig. 3). Gelangen nun die Bajonett-Zapfen 7 an die Rastnocken 11, so wird der Dichtring 6 noch weiter komprimiert und der Konus 5 in den Konus 4 völlig hineingeschoben, bis beide Konen 4, 5 fast völlig zur Anlage gelangen. Dieser fast völlige oder völlige Anlagezustand ist aber unerwünscht, da ein solcher stetiger mechanischer Verspannungszustand in Verbindung mit thermischen, mechanischen und chemischen Beanspruchungen zur vorzeitigen Zerstörung und Bruch der Kupplungsteile 2, 3 führen kann. Vielmehr werden die Bajonett-Zapfen 7 nach Überwindung der Rastnocken 11 in eine pfannenförmige Vertiefung 8b, welche in Fortsetzung der Bajonett-Kurven-Nuten 8 liegen, zurückgedrückt und zwar mit Hilfe der Kompressionsrückstellkraft des Dichtringes 6. Hierdurch entsteht ein definierter Spalt 19 zwischen den Konen 4 und 5 (Fig. 3). Durch diesen definierten Spalt 19 wird ein thermischer bzw. mechanischer Spannungsausgleich für jedes Kupplungsteil 2 und 3 separat möglich. Eine Anlage besteht lediglich im Bereich des elastischen Dichtungsringes 6, welcher damit für sich allein die volle Dichtwirkung aufbringt.

In Fig. 3 und 4 sind die beiden Saugschläuche 17 und 18 dargestellt, wobei der Saugschlauch 17 zur Wunde des Patienten und der Saugschlauch 18 zum Aufnahmegefäß führt. Der Saugschlauch 17 ist auf den Schlauchstutzen 12 aufgeschoben und wird gegen unbeabsichtigtes Lösen durch einen gestuften Konus 12a in axialer Richtung gehalten. Das Lumen des Stutzens 12 ist mit 13 bezeichnet. Im männlichen Kupplungsteil 3 ist ein Konus 21 zur Aufnahme des Saugschlauches 18 angeordnet, in welchen dieser vorzugsweise eingeklebt werden kann. Das Lumen des Kupplungsteiles 3 ist mit 14 bezeichnet.

An den beiden Kupplungsteilen sind Griff-Flügel 15 und 16 angeordnet, welche die Handhabung des Schlauchverbinders insbesondere deren Verdrehen zueinander beim Kuppeln, erleichtert.

Die beiden Kupplungsteile 2 und 3 sind vorzugsweise aus thermoplastischem, transparentem Kunststoff gefertigt.

## Patentansprüche

1. Schlauchverbinder für Vakuumdrainagen, bestehend aus einem weiblichen Kupplungsteil (2) mit Innenkonus (4), einem männlichem Kupplungsteil (3) mit Außenkonus (5) mit gleicher Konizität, wobei der Außenkonus (5) eine Nut (6a) mit einem elastischen Dichtring (6) aufweist, und einer Verbindungseinrichtung, **dadurch gekennzeichnet, daß** als Verbindungseinrichtung ein Bajonett-Verschluß vorgesehen ist, der durch zwei Bajonett-Zapfen (7) am männlichen Kupplungsteil (3), die mit zwei Bajonett-Kurvennuten (8) des weiblichen Kupplungsteiles (2) korrespondieren, gebildet wird, und der elastische Dichtring (6) die Dichtwirkung zwischen den beiden Kupplungsteilen (2, 3) in verbundenem Zustand bewirkt, und der Bajonettverschluß (7, 8) so dimensioniert ist, daß beim Verbinden der Kupplungsteile (2, 3) zwischen Innenkonus (4) und Außenkonus (5) ein definierter Spalt (19) entsteht.

## Claims

1. Hose connector for vacuum drainages, comprising a female coupling member (2) with an inner cone (4), a male coupling member (3) with an outer cone (5) of the same conical shape, the outer cone (5) having a groove (6a) with an elastic sealing ring (6), and a connecting means, **characterized in that** a bayonet lock is provided as the coupling means, formed by two bayonet pins (7) at the male coupling member (3) which pins correspond to two curved bayonet grooves (8) of the female coupling member (2), and the elastic sealing ring (6) provides for the sealing effect between the two coupling members (2, 3) in the connected state, and the bayonet lock (7, 8) is dimensioned such that a defined gap (19) is formed between the inner cone (4) and the outer cone (5) upon connecting the coupling members (2, 3).

## Revendications

1. Raccord de tuyau souple pour des drainages sous vide, composé d'un élément d'accouplement femelle (2) doté d'un cône interne (4), d'un élément d'accouplement mâle (3) doté d'un cône externe (5) de même conicité, le cône externe (5) présentant une rainure (6a) avec une bague d'étanchéité (6) élastique, et d'un dispositif de liaison,
**caractérisé en ce que** :
une fermeture à baïonnette est prévue pour servir de dispositif de liaison, laquelle est formée par deux goupilles de baïonnette (7) sur l'élément d'accouplement mâle (3), qui correspondent avec deux rainures courbes de baïonnette (8) sur l'élément d'accouplement femelle (2), la bague d'étanchéité élastique (6) produisant l'effet d'étanchéité entre les deux éléments d'accouplement (2, 3) à l'état relié, et la fermeture à baïonnette (7, 8) étant dimensionnée de manière à obtenir un écartement (19) déterminé entre le cône interne (4) et le cône externe (5) lorsque les éléments d'accouplement (2, 3) sont reliés.
